Europäisches Patentamt

European Patent Office

Office européen des brevets

(11) Publication number: **0 501 321 A1**

# EUROPEAN PATENT APPLICATION

(21) Application number: **92102898.1**

(22) Date of filing: **21.02.92**

(51) Int. Cl.5: **C07K 15/00, A61K 39/395**

(30) Priority: **26.02.91 JP 30748/91**
**26.08.91 JP 213451/91**

(43) Date of publication of application:
**02.09.92 Bulletin 92/36**

(84) Designated Contracting States:
**AT BE CH DE DK ES FR GB GR IT LI LU MC NL PT SE**

(71) Applicant: **Hirasawa, Keisuke**
**12-11 Nakajuku**
**Itabashi-ku, Tokyo-to(JP)**

(72) Inventor: **Hirasawa, Keisuke**
**12-11 Nakajuku**
**Itabashi-ku, Tokyo-to(JP)**

(74) Representative: **Türk, Dietmar, Dr. rer. nat.**
**Türk, Gille + Hrabal Patentanwälte**
**Brucknerstrasse 20**
**W-4000 Düsseldorf 13(DE)**

(54) **Immunosuppressive drug containing an anti-estradiol antibody.**

(57) An immunosuppressive drug comprising an anti-estradiol antibody as an effective ingredient. The drug of the present invention has a clear immunosuppressive activity and does not have serious side effects or toxicity.

FIG.1

The present invention relates to an immunosuppressive drug.

An immune system is one of self-defence actions of living creatures. Owing to the immune system, hosts discriminate between self and not-self substances such as viruses or bacteria which should be excluded from own bodies.

However, this beneficial immune system occasionally works negatively to hosts, appeared as a rejection of an allogeneic transplanted grafts and a production of an antibody against own body or organs in autoimmune disorders.

Immunosuppressive drugs are used for the suppression of such unfavorable immune responses such as rejection against the transplanted organs and the production of auto-antibodies.

Although clinically usable immunosuppressive drugs such as Cyclosporine which suppress lymphocyte blast formation by stimulation of foreign antigens or lectins have been introduced for the treatments of rejection in organ transplantation and autoimmune diseases, these immunosuppressive drugs are not effective enough or possessing severe side effects on the patients and there are some differences in the effectiveness of the drugs among individuals, especially females are unsusceptible to immunosuppressive treatments (ref. Hirasawa K., Enosawa S., Transplant Proc., Sex differences in the prolongation of allogeneic skin graft survival in rats treated with Cyclosporin A: Effects of orchiectomy and pregnancy. Vol 21, pp 881-884, 1989).

An introduction of novel immunosuppressive drugs having simple and elegant efficacy has been expected for the treatments of allograft rejections and autoimmune diseases such as systemic lupus erythematosus and rhumatoid arthritis.

An immunosuppressive drug comprising an antiestradiol antibody of the present invention has been discovered from a series of studies on questions, (1) why female patients reject human kidney grafts quicker than males (ref. Terasaki PI, Mickey MR, Cecka M, Cicciarelli J, Cook D, Iwasaki Y Toyotomi A Wang L, Clinical Transplants, Overview, pp 467-490, 1987) (2) why females are more resistant to viruses and bacterial infections than males (ref. London WT, Drew JS, Proc. Natl. Acad. Sci., Sex differences in hepatitis B infection among patients receiving cronic dialysis treatment., Vol 74, pp 2561-2563, 1977) (ref. Wheater DWF, Hurst EW, J. Pathol Bact., The effect of sex on bacterial infections and on the chemotherapy of one of them. Vol 82, pp 117-130, 1961) and (3) why females are more sufferring from autoimmune diseases than males (ref. Inman RD, Arthritis Rheumatism, Immunologic sex differences and the female predominance in systemic lupus erythematosus. Vol 21 pp 849-852, 1978).

These phenomena suggest that females have stronger and longer lasting immune responses than males. Previous studies showed that exogenous female sex steroid hormone, especially estradiol, reversed immunosuppressive activity of a non-specific immunosuppressive drug Cyclosporine as appreared in acceleration of the graft rejection (ref. Hirasawa K, Enosawa S, Transplantation, Effects of sex steroid hormones on sex-associated differences in the survival time of allogeneic skin grafts rats. Vol. 50, pp 637-64, 1990) (ref. Hirasawa K, Kamada N, Transplant. Proc., Female sex hormone, estradiol antagonizes the immunosuppressive activity of Cyclosporine in rat organ transplantation. Vol 24, pp 408-409, 1992). Also, exogenous estradiol enhanced production of antibody against bacteria (ref. Kenny JF, Pangburn PC, Trail G, Infect. Immun., Effect of estradiol on immune competence: in vivo and in vitro studies. Vol 13, pp 448-456, 1976).

However, as to endogenous estradiol, an action on immune system in hosts is not at all known hitherto.

An object of the present invention is to provide an immunosuppressive drug which has low side effects and is useful for immunosuppressive treatment of organ transplantations and autoimmune diseases.

This and other objects of the present invention will become apparent from the description hereinafter.

It has now been found not only that endogenous estradiol potentiates host's immunity, but also that antibodies against estradiol surprisingly possess immunosuppressive activity in long periods without obvious side effects, as appeared that the antibody prolonged the survival time of the fully allogeneic skin graft with or without non-specific immunosuppressive drug, Cyclosporine.

According to the present invention there is provided an immunosuppressive drug comprising an anti-estradiol antibody as an effective ingredient.

Figure 1 is a graph showing effects of various amounts of anti-estradiol monoclonal antibody on the survival time of the skin graft in recipients treated with non-specific immunosuppressive drug, Cyclosporine in Example 3.

Figure 2 is a graph showing the relationship between percentage of the graft survival and days after the operation in Example 3.

Anti-estradiol antibodies which are active ingredients of the present invention can be prepared by any usual methods. The antibodies can be antisera (polyclonal antibodies), monoclonal antibodies against estradiol or the like including anti-idiotype.

For example, antisera, polyclonal antibodies against estradiol are taken from sera of animals such as rats immunized with bovine serum albumin conjugated estradiol-6-(O-carboxymethyl oxime). For example, monoclonal antibodies against estradiol are produced from clones made by hybridization of non-immunogloblin-producing myeloma cells of mice or the like and spleen cells of the animals immunized as above.

The immunosuppressive drug of the present invention can be administered by parenteral administration such as intravenous administration, subcutaneous administration or intramuscular administration. The administration route is not particularly limited and can be suitably selected according to the purpose of the treatments.

The immunosuppressive drug of the present invention can be used in various preparation forms such as injection, transfusion solution and eye drops containing about 0.1 to about 3 % by weight of an anti-estradiol antibody. These various preparations can be prepared in usual methods by using conventional additives which are generally usable in the field of pharmaceutical preparation such as excipient, binder, solvent, solubilizer, emulsifier or suspending agent, according to the purpose of the treatment.

As such additives, there are, for example, lactose, sucrose, glucose, starch, crystalline cellulose, hydroxypropylcellulose, carboxymethylcellulose, arabic gum, gelatin, magnesium alumino meta silicate, anhydrous calcium phosphate, citric acid, trisodium citrate, hydroxypropylcellulose, sorbitol, sorbitan esters of fatty acids, polyvinylpyrrolidone, vegetable oil such as peanut oil or olive oil, benzyl alcohol, propylene glycol, water and the like.

The effective dosage of the immunosuppressive drug of the present invention for intravenous injection can be chosen from a range of 0.1-50 mg of anti-estradiol monoclonal antibody/day as an effective ingredient for adults.

The immunosuppressive drug of the present invention is effective on the treatments of rejection in organ transplantations and autoimmune diseases such as systemic lupus erythematosus and rheumatoid arthritis.

Also the immunosuppressive drug of the present invention is synergistically effective with the non-specific immunosuppressive drugs such as Cyclosporine, "FK506" (Fujisawa Pharmaceutical Co. Ltd., Japan) and Rapamysin which have been introduced for clinical use. Those conventional drugs surppress the lymphocyte blast formation stimulated by foreign antigens or lectins such as concanavalin A. When the immunosuppressive drug of the present invention was used as a combination therapy with those drugs stated above, the treatment of the immunosuppressive drug of the present invention can be performed at any time. There is no injection priority between conventional drugs and the immunosuppressive drug of the present invention and there is no restriction in intervals of the administration of them, but preferably at 24 hours intervals.

The immunosuppressive drug of the present invention is more specifically described and explained by means of the following Examples. It is to be understood that the present invention is not limited to the examples, and various changes and modifications can be made in the invention without departing from the spirit and scope thereof.

Reference Example

Preparation of anti-estradiol monoclonal antibody

Anti-estradiol monoclonal antibody (rat origin, clone No. 2F9) in ascites of rats was purchased from Intra Pharm laboratories Ltd., Israel (ref. Kohen P, Lichter S, Serono Symposia Publications, Monoclonal antibodies to steroid hormones, Vol 30, pp87-95, 1986). The ascites were centrifuged and diluted ten times with 15 mM, pH 7.0 phosphate buffer solution. Then the solution of the ascites was applied on the hydroxylapatite column (0.7 $cm^2$ x 20 cm) equilibrated with 15mM, pH 7.0 phosphate buffer solution. The elution procedures were carried out by stepwise elutions with from 15 mM to 300 mM phosphate buffer solutions as described previously (ref. Bukovsky J, Kennett RH, Hybridoma, Simple and rapid purification of monoclonal antibodies from cell culture supernatants and ascites fluids by hydroxylapatite chromatography on analytical and preparative scales, Vol. 6, pp219-228, 1987). Then the biochemical and immunological characterizations of thus purified antibodies were determined as follows:

Anti-estradiol monoclonal antibody

(1) Specific activity: 330 pico moles estradiol/mg protein
(2) Antibody Isotype: IgG$_{2a}$

(3) Cross reactivity

| Steroids | Cross reactivity (%) |
|---|---|
| Estradiol | 100 |
| Estriol | < 0.1 |
| Estrone | < 0.1 |
| Progesterone | < 0.1 |
| Cortisol | < 0.1 |
| Testosterone | < 0.1 |

Example 1

Preparation of injection solution

The injection solution of the anti-estradiol monoclonal antibody was prepared as the following formation.

| Anti-estradiol monoclonal antibody Saline | 3 mg balance |
|---|---|
| Total | 1 mℓ |

Example 2

Effect of anti-estradiol monoclonal antibody on the survival time of the skin graft (1)

Fully allogeneic inbred rat strains, PVG-RT1a rats (donor) and PVG-RT1c rats (recipient) were obtained from Harlan Olac Ltd., U.K.

Sexually mature adult male rats, 12-15 weeks old, (body weight, 250-290 g) were used for the experiments of skin grafting. Six recipients were used in each group.

Full thickness of skin graft (3 x 3 cm) from ventral trunk of adult male PVG-RT1a rats were grafted in adult male PVG-RT1c rats according to the usual method (ref. Towpic E, Kupiec-Weglinski JW, Schneider TM, Tyler D, Padberg W, Araneda D, Tilney NL, Transplantation, Cyclosporine and experimantal skin allograft. II. In definite survival and development of specific immunogic unresponsiveness, Vol. 40, pp714-718, 1985).

The rejection of skin grafts were determined as total epithelial necrosis.

The skins from PVG-RT1a rats in normal recipients of PVG-RT1c rats survived for 7.3 ± 0.5 days (Mean survival day ± SD, hereinafter the same). Another six recipients were administered with anti-estradiol monoclonal antibody obtained in Reference Example (0.38 mg in 1 mℓ of saline) within six hours after the grafting. The survival time of the grafts in the antibody administered recipients was prolonged to 9.8 ± 1.7 days. The difference in the survival times between two groups, the antibody administered group and not administered group was statistically significant at $p = 0.0007$ under Student T test.

The results presented here showed that anti-estradiol monoclonal antibody has an immunosuppressive activity.

Example 3

Effect of anti estradiol monoclonal antibody on the survival time of the skin graft (2)

Fully allogeneic inbred rat strains, DA-RT1a rats (donor) and PVG-RT1c rats (recipient) were obtained from Harlen Olac Ltd., U.K. The sexually mature male rats, 12-15 weeks old, (body weight, 250-290 g) were used for the experiments of the skin grafting. Six recipients were also used in each group A to E.

The skin grafting methods were the same as described in Example 2. Cyclosporine (trade name: Sandimmun, Sandoz, Basel, Swizerland) was used as a non-specific immunosuppressive drug and administered intramuscularly into rear legs (20 mg/kg/day) within six hours after the operation and thereafter at every 24 hour for two weeks.

The anti-estradiol monoclonal antibody in Reference Example was also used here.

The effect of the anti-estradiol monoclonal antibody on the survival time of the skin grafts was examined in the presence of a non-specific immunosuppressive drug, Cyclosporine. Recipients were differently treated as follows:

| Groups: Post-operative treatments (Pre-operative treatments can be also done.) | |
|---|---|
| A group: | Cyclosporine only |
| B group: | Cyclosporine + the antibody (0.38 mg in 1 mℓ of saline) |
| C group: | Cyclosporine + the antibody (0.75 mg in 1 mℓ of saline) |
| D group: | Cyclosporine + the antibody (1.5 mg in 1 mℓ of saline) |
| E group: | Cyclosporine + the antibody (3.0 mg in 1 mℓ of saline) |
| F group: | Olive oil (1 mℓ /mg/day) + 1 mℓ of saline |

The administrations of the solution of the antibody (1 mℓ /rat) were carried out on the day of the operation only.

The recipients in F group were treated with olive oil and saline in place of Cyclosporine and the antibody, respectively.

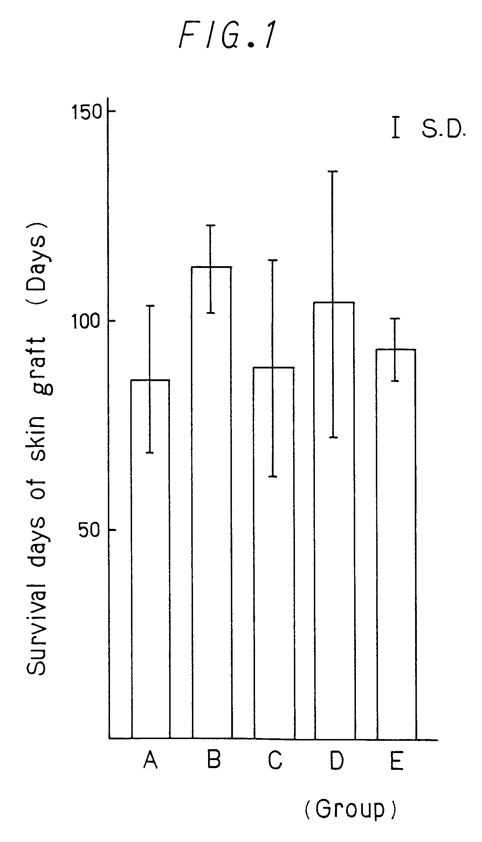

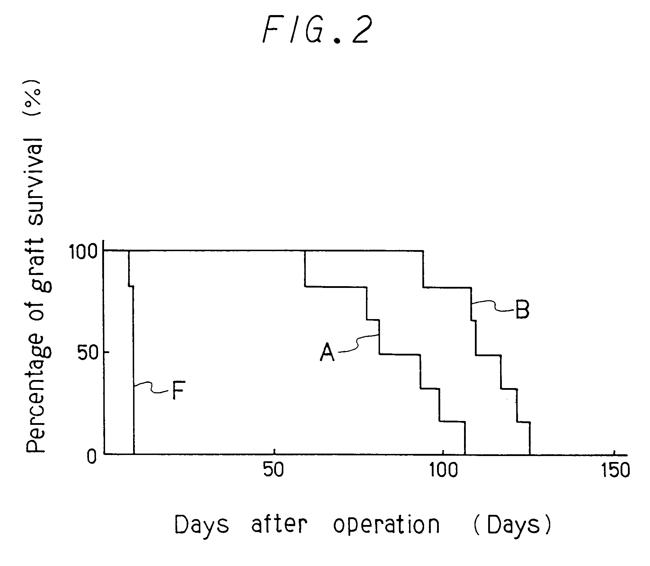

The results are shown in Figures 1 and 2.

Figure 1 shows effects of various amounts of anti-estradiol monoclonal antibody on the graft survival days in the presence of Cyclosporine. As shown in Figure 1, the survival times of the grafts in the recipients treated with both anti-estradiol monoclonal antibody and Cyclosporine were prolonged more than those in the recipients treated with only Cyclosporine. The optimun synergistic immunosuppressive effect of anti-estradiol monoclonal antibody was indicated at a low dosage of about 0.38 mg/rat administration.

Figure 2 shows a synergistic immunosuppressive effect of the anti-estradiol monoclonal antibody with Cyclosporine on the prolongation of the survival time of the skin graft expressed by the comparison with respect to the days of the graft survival and percentage of the graft survival.

The difference in the survival times of the grafts between A group (only Cyclosporine administered) and B group (Cyclosporine + anti-estradiol monoclonal antibody 0.38 mg administered) was statistically significant at $p = 0.0001$ under Student T test.

As shown here, immunosuppressive activity of the anti-estradiol monoclonal antibody was clearly demonstrated.

Example 4

Acute Toxicity test

Male PVG-RT1c rats (8 weeks old, body weight 200-220 g) were administered intravenously with the injection solution of the anti-estradiol monoclonal antibody obtained in Reference Example (10 mg/mℓ saline). The values of the $LD_{50}$ were not less than 40 mg/kg.

In addition to the ingredients used in the Examples, other ingredients can be used in the Examples as set forth in the specification to obtain substantially the same results.

**Claims**

1. An immunosuppressive drug comprising an anti-estradiol antibody as an effective ingredient.

2. The drug of Claim 1 comprising an anti-estradiol antibody and pharmaceutically usable carrier or additive.

3. Use of an anti-estradiol antibody as an effective ingredient for suppressing fundamental immune responses.

4. Use of an anti-estradiol antibody for manufacture of medicament for suppressing fundamental immune responses.

# FIG.1

# FIG.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | MEDLINE Abstract number: 91118507 HIRASAWA K. et al:"Sex-associated differences in organ transplantation: different effects of steroid hormones, testosterone, estradiol progesterone, and prednisolone on the survival time off allogeneic skin graft in rats treated with cyclosporine A." & TRANSPLANT PROC., February 1991, 23 , p 714-715. | 1-4 | C07K15/00 A61K39/395 |
| X D | MEDLINE Abstract number: 91020368 HIRASAWA  K. et al: " Effects of sex steroid hormones on sex-associated differences in the survival time of allogeneic skin grafts in rats" Evidence that testosterone enhances and estradiol reverses the immunosuppressive activity of cyclosporine. & TRANSPLANTATION, October 1990, 50 (4), p 637-41 | 1-4 | |
| X | MEDLINE Abstract number: 89326493 CARLSTEN H. et al: "Oestradiol suppression of delayed-type hypersensitivity in autoimmune (NZB/NZW) F1 mice is a trait inherited from the healthy NZW parental strain." & IMMUNOLOGY , June 1989, 67(2), p 205-9 | 1-4 | **TECHNICAL FIELDS SEARCHED (Int. Cl.5 )** A61K G01N C07K |
| A | MEDLINE Abstract number: 78241146 FEIGEN G.A. et al: " Sex hormones and the immune response.II. Perturbation of antibody production by estradiol 17 beta." & INT. ARCH. ALLERGY. APPL. IMMUNOL. 1978, 57(6), p 488-97 | 1-4 | |
| A | GB-A-2 197 588 (KUREHA KAGAKU KOGYO K.K.) * abstract * | 1-4 | |

-/--

The present search report has been drawn up for all claims

| Place of search | Date of completion of the search | Examiner |
|---|---|---|
| BERLIN | 06 MAY 1992 | AVEDIKIAN P.F. |

EPO FORM 1503 03.82 (P0401)

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | JAPANESE PATENTS GAZETTE<br>Derwent Publications Ltd., London, GB;<br>& JP-A-1 109 262 (EIKEN KAGAKU K.K.) 26 April 1989<br>* abstract *<br><br>----- | 1-4 | |
| | | | TECHNICAL FIELDS SEARCHED (Int. Cl.5 ) |

The present search report has been drawn up for all claims

| Place of search | Date of completion of the search | Examiner |
|---|---|---|
| BERLIN | 06 MAY 1992 | AVEDIKIAN P.F. |

EPO FORM 1503 03.82 (P0401)